# EUROPEAN PATENT SPECIFICATION

(11) **EP 0 999 786 B1**
(45) Date of publication and mention of the grant of the patent: **27.11.2002**
(21) Application number: 98936493.0
(22) Date of filing: 03.08.1998
(51) Int. Cl.: A61B 5/15, C12Q 1/54

(54) **DEVICE FOR TESTING ANALYTE CONCENTRATION IN A FLUID**
VORRICHTUNG ZUM TESTEN DER ANALYTKONZENTRATION IN EINER FLÜSSIGKEIT
DISPOSITIF POUR TESTER UNE CONCENTRATION D'ANALYSAT DANS UN FLUIDE

(30) Priority: 01.08.1997 GB 9716254
(43) Date of publication of application: 17.05.2000
(73) Proprietor: HYPOGUARD LIMITED, Woodbridge Suffolk IP12 1PE (GB)
(72) Inventor: ALTON, Robin, Essex CM17 0JU (GB); BLACK, Murdo, MacPhee, Oxfordshire OX7 6LE (GB); HO, Wah, On, Newcastle uponTyne NE6 2RP (GB); STECKERL, Lorna, Suffolk IP6 0MP (GB); WANG, Yuan, Warrington, Cheshire WA4 2QS (GB); MENEZES, Joel, Withington, Manchester M20 1HX (GB); TRELOAR, Paul, Chester CH3 7PQ (GB)
(74) Representative: Gemmell, Peter Alan, Dr.
(86) International application number: GB9802184
(87) International publication number: WO99005966

(56) References cited:
- EP-A1- 0 247 850
- GB-A- 2 159 625

## Description

### FIELD OF THE INVENTION

The present invention relates to a device for point of care testing of analyte concentrations in a fluid, notably a biological fluid. The device is particularly for use in testing blood for an analyte such as glucose or cholesterol.

### BACKGROUND TO THE INVENTION

Diabetics need to test their blood glucose levels frequently, often several times a day. This needs to be done accurately, consistently and with the minimum of inconvenience to the diabetic. The testing is typically carried out using a point of care test strip and meter; that is, the diabetic takes test readings wherever he happens to be, using a portable meter system.

At present there is no commercially available non-invasive test method. All current systems rely on a blood sample being placed on a test strip which reacts with glucose in the blood. This reaction can then be read by some method, for example comparing a generated colour with a colour chart, or by using a dedicated meter. Reading a colour chart by eye can be inaccurate, particularly where the diabetic suffers from imperfect colour visual acuity.

All current point of care meter systems use disposable test strips in increasingly sophisticated meters. These tend to offer increased real-time memory and may be downloadable to a PC. Some offer more reliable results by providing a coding chip in each pot of strips supplied. The coding chip carries information about the batch of strips which is used to calibrate the meter by the user before readings are taken using the strips. The advantage this has for a point of care test is that it reduces the chance of an inaccurate result being obtained because the wrong calibration code was entered. However, the patient still has to insert a coding chip.

The patent document WO-9703355 discloses a portable apparatus for testing analyte concentration comprising a removable test member provided with sensing means and meter provided with display means. This apparatus is complex to manufacture and use and must be operated by a skilled operator rather than by the patient.

It is an object of the present invention to provide a point of care test device which is convenient and easy to use, and which reduces the possibility of inaccurate results due to operator error.

### SUMMARY OF THE INVENTION

According to an aspect of the present invention there is provided a device for point of care testing of analyte concentration in a fluid applied thereto, the device comprising a test member provided with sensing means adapted to produce an electrical signal in response to the concentration of analyte in an applied fluid, and a meter comprising electronics means for producing a signal output which is dependent on the electrical signal from the test member, the meter being provided with display means for displaying the signal output; characterised in that the test member is re-usable and permanently secured in relation to at least a part of the meter.

By providing a re-usable test member permanently attached to at least part of the meter, the device combines the accuracy of a meter with the ease of use of a visual system. Typically, the test member will be a test strip which has dimensions similar to a conventional test strip. For convenience hereinafter the invention will be described with reference to a test member which is a test strip.

The meter may be integrally formed with the test strip.

The device may be used to test for any analyte, but it is particularly preferred that it is used for testing for the concentration of glucose in blood. For convenience the invention will be described hereinafter with reference to this application, but it is to be understood that the invention is not limited to this embodiment.

The device may be discarded after a predetermined number of tests, for example after 50 tests. This would provide a diabetic with a simple, convenient, accurate and consistent test.

The test strip could be permanently attached to electronic calibration means of the meter, to permit factory calibration of the signal output from the meter in response to electric current or other electrical signal generated by the sensing means when a known concentration of analyte is applied to the test strip. Preferably, the test strip is permanently attached to the whole meter.

Because the test strip is attached to and sold with the meter or the calibration electronics means, the whole unit can be pre-calibrated at the factory. This avoids the need for the user to perform a calibration operation on a meter system before starting to use a new batch of test strips. The user may optionally recalibrate the meter at regular intervals, for example each week, and a control solution may be provided with the meter or separately, for carrying out a calibration. Another aspect of the invention therefore provides a test kit for point of care testing, comprising a device as described herein, and a control solution for use in calibrating the meter of the device.

The device may be provided with a port, for example for communication with or downloading to or from a computer. This port could be used during calibration in the factory, for example to take into account factors specific to the batch of test strips, and optionally also factors such as temperature, relative humidity, and altitude of the intended destination. The port could also be used to record test results on a user's PC.

The sensing means preferably comprises one or more reagents which react with the glucose to produce at least one electroactive species, and an electrical circuit which passes a current the magnitude of which varies according to the concentration of applied glucose. Such sensing means will be well known to those skilled in the art.

The test strip may be re-usable by being washable, for example in tap water, to remove unreacted species and protinaceous material which would foul the electrode surface and/or any covering membrane. Alternatively the test strip could be re-usable by progressive removal of layers or strips which carry the reagents and the conductive species when generated.

The test strip preferably uses an immobilised enzyme system, for example glucose oxidase ("GOD") or glucose dehydrogenase immobilised, preferably by physical adsorption, onto platinised or rhodiumised carbon. The use of immobilised enzymes is described in US Patent No. 4,970,145 and US Patent No. 5,122,456.

To facilitate cleaning of the test strip and to minimise sample volumes required, it is particularly preferred that the area containing the working electrode and enzyme is covered by a semi-permeable membrane. The membrane must, of course be permeable to the analyte and oxygen. Test strips of this general type are known, and are described in EP 0 690 134, for example.

To reduce the effect of background interferences from electroactive species such as ascorbate, urate, and acetaminophen (paracetamol), it is preferred that in addition to an active electrode which contains GOD, a similar dummy electrode is provided which contains an inert non-enzymic protein, for example bovine serum albumin ("BSA"). The active electrode will, in addition to responding to background electroactive species, respond specifically to the presence of glucose in the sample. The subtraction of the dummy electrode response from the active electrode response will give an analytical response due specifically to the presence of glucose in the sample.

In a preferred embodiment, the test strip is provided with a cap which covers the area to which blood is applied, the inside surface of the cap being provided with means for wiping or washing the test strip when the cap is removed from and/or replaced on the test strip. The cap could alternatively abrade or strip off one or more layers on the test strip when removed from and/or replaced on the test strip.

In a particularly preferred embodiment, each instance when the cap is removed and replaced is recorded on a counter. When the cap has been removed and replaced a predetermined number of times, a locking mechanism is actuated to prevent further removal of the cap. The user may thereby be prevented from re-using the device beyond its intended life.

The counter may be mechanical or, preferably, an electronic memory.

The device is preferably compact and discreet, for example it may be shaped like a pen, or flat and rounded, to fit in a pocket.

Although the test member of the device may be washed under a tap to clean it, other cleaning means could optionally be provided in the device. For example the device may include a housing in which is located a reservoir of washing fluid, and means for releasing a metered quantity of washing fluid onto the sample area of the test strip when desired. This would permit a user to clean the test strip when an external source of cleaning fluid such as a tap is not readily available.

The meter is preferably permanently attached to the display means, but it would also be possible for the electronics which are used to calibrate the test strip in the factory, and optionally a counter, to be housed in a cartridge which is permanently attached to the test strip, the cartridge being releasably secured to the display means. This arrangement would permit a single display means to be used with a number of different disposable cartridges, each of which could carry a different analyte detection system. The benefit of pre-calibration of the test member and meter in the factory would be retained.

Accordingly, another aspect of the invention provides a cartridge comprising a housing which is permanently attached to a test member for point of care testing of analyte concentration in a fluid applied thereto, the test member and/or the housing provided with sensing means adapted to produce an electrical signal in response to the concentration of analyte in a fluid applied to the test member, and the housing provided with electronic means for calibrating the test member, the cartridge being provided with mounting means for releasably connecting the cartridge to display means for displaying an output reading which is dependent on the electrical signal.

The releasable connecting means may comprise a conventional edge connector or a plug or socket for mating with a suitable corresponding structure on the display means.

Where a counter is housed in the device, it may be programmed to cause something to happen when a preset number of readings have been taken. For example, the display means may be de-activated, or a warning light may come on to warn the user to discard the device. The counter may be activated by having the user push a button to get a display reading, each push of the button incrementing the counter by one. Alternatively, the counter may be incremented each time the sensor detects an initial rate of change in current from the active electrode which corresponds with human blood but not with calibration or washing solution.

### BRIEF DESCRIPTION OF THE DRAWINGS

The invention will now be further described, by way of example, with reference to the following drawings in which:
Figure 1 shows a device for point of care testing of analyte concentration in a fluid, in accordance with the present invention;
Figure 2 shows a cartridge and display device for point of care testing in accordance with another aspect of the present invention;
Figure 3 is a graph of current v glucose concentration for an electrode suitable for use in manufacturing a device in accordance with the present invention;
Figure 4 is a graph similar to Figure 3, in which the electrode has a PVC membrane;

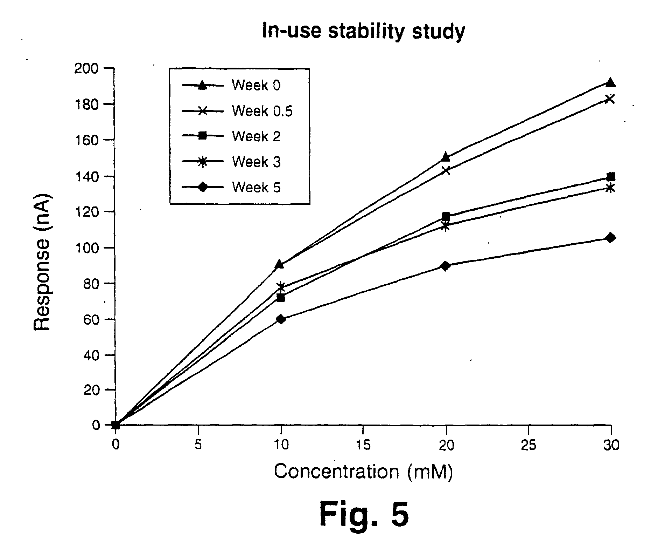

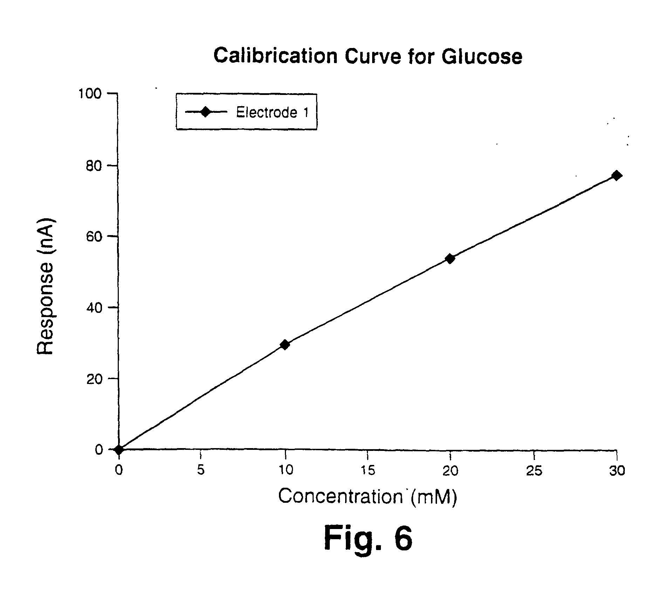

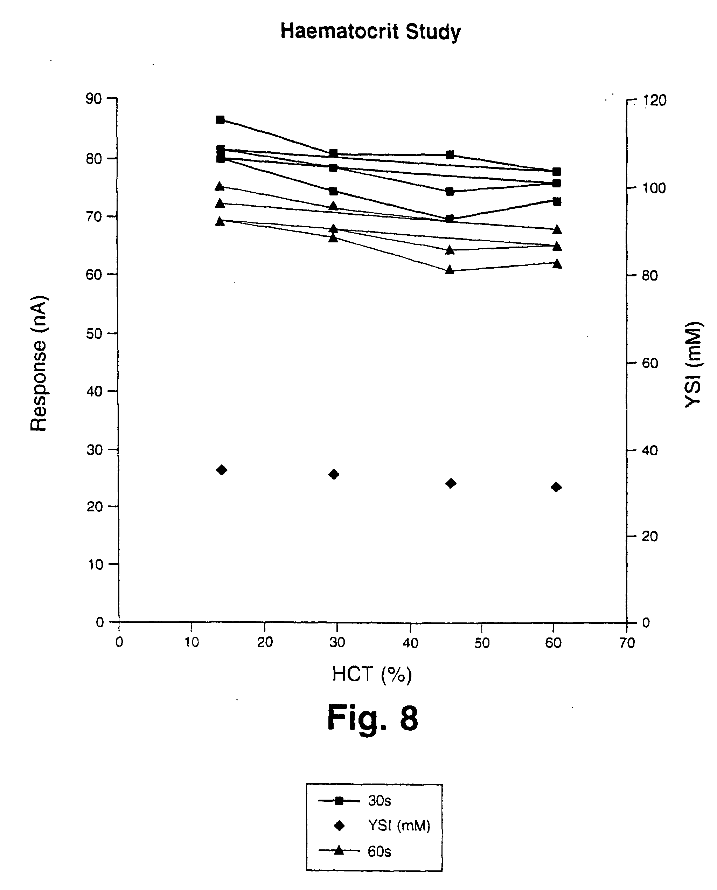

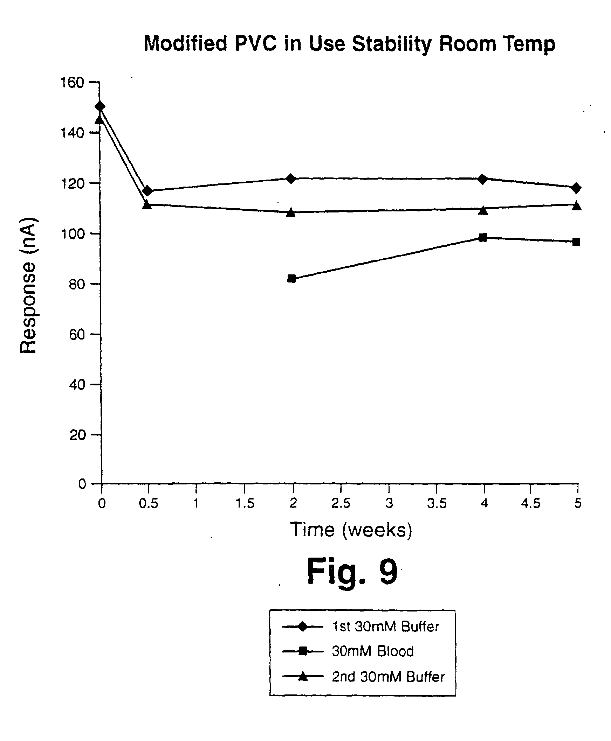

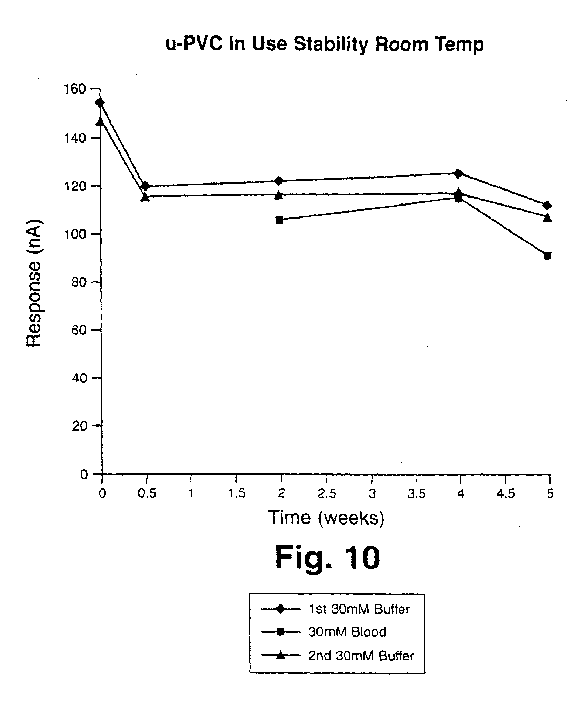

Figures 5 to 10 are graphs of various test results for test strips suitable for use in the present invention having alternative electrode formulations using a different conductive ink;

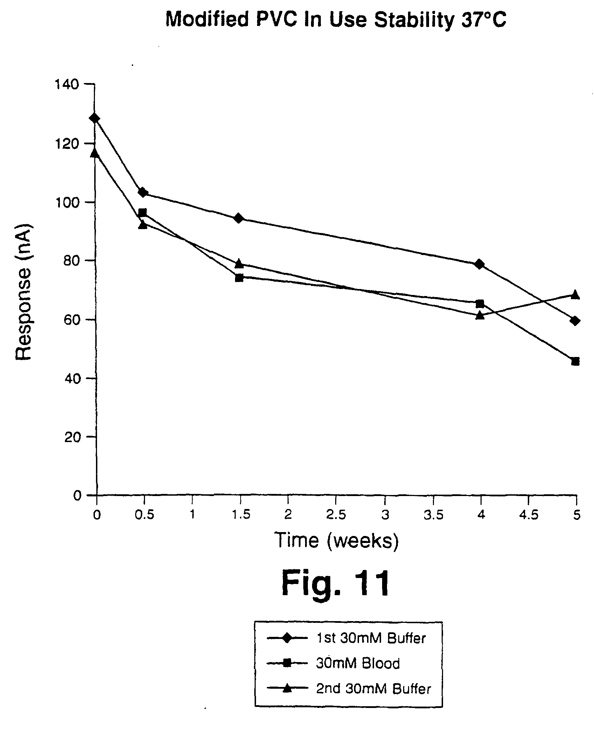

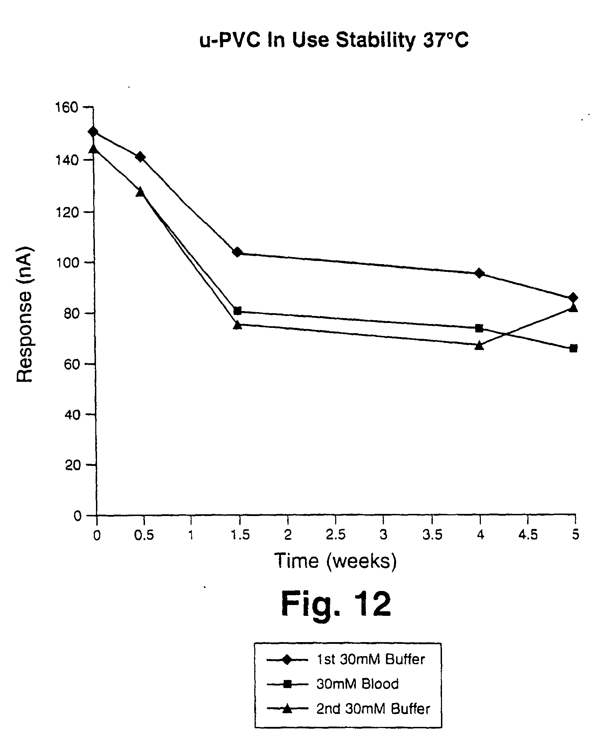

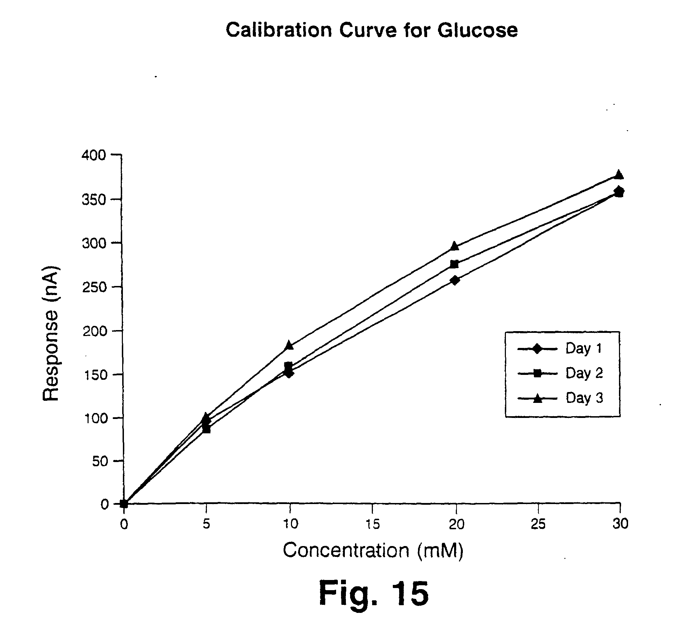

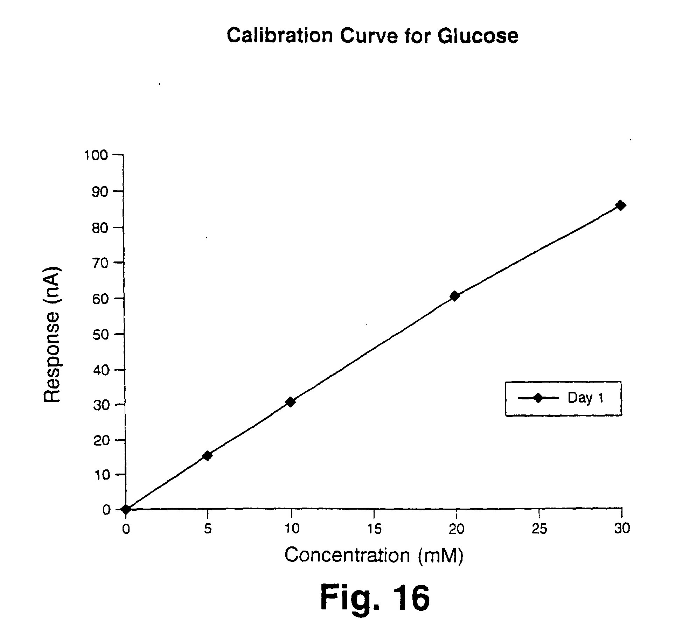

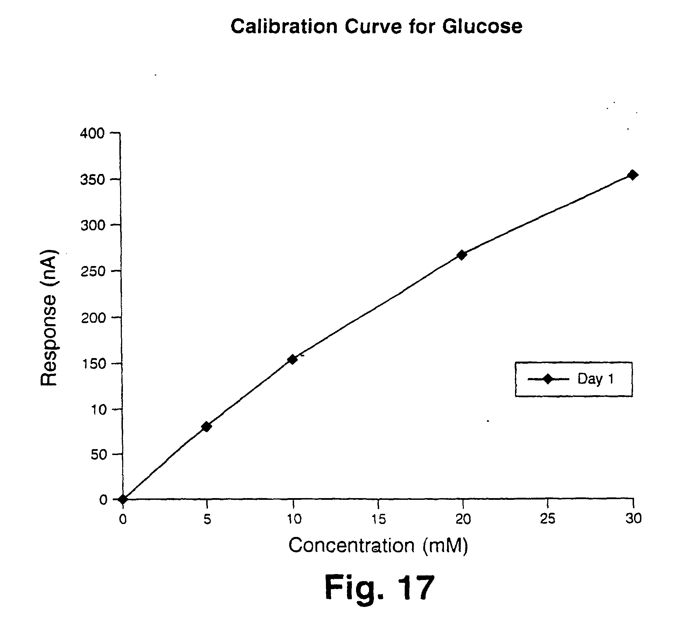

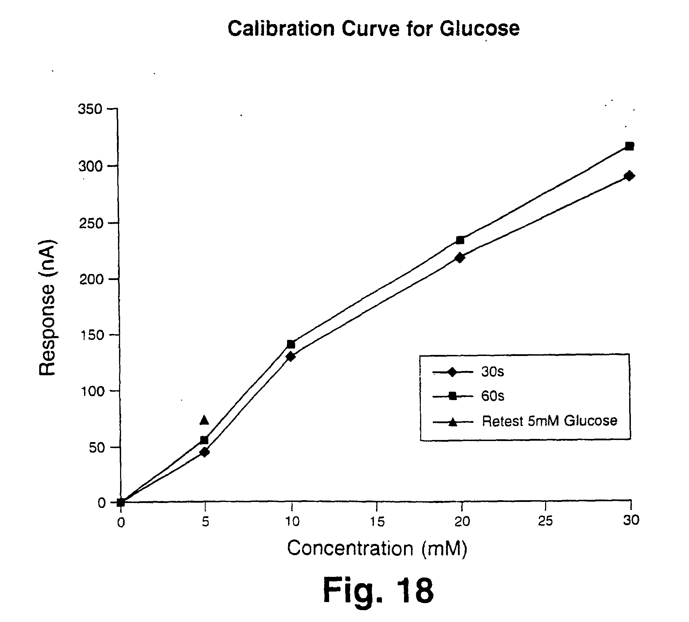

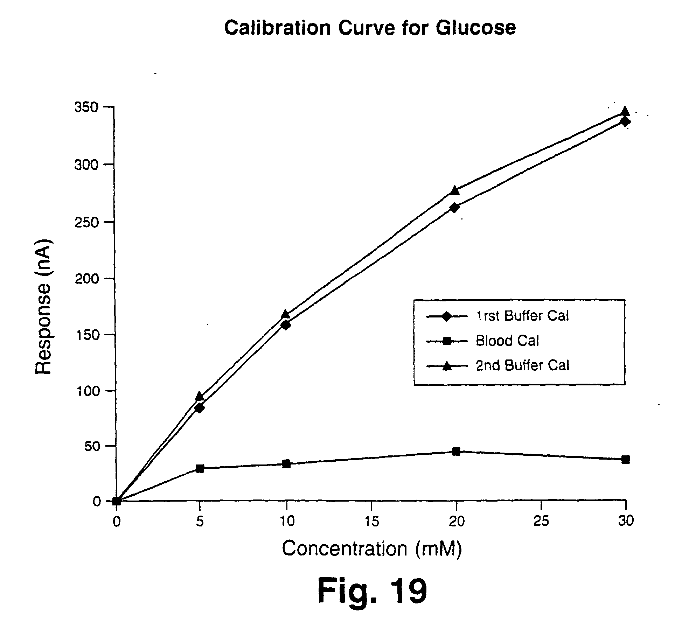

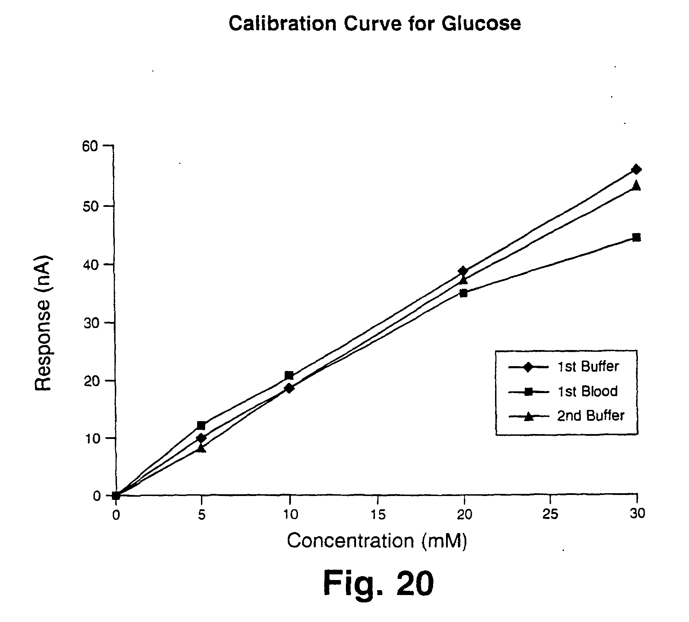

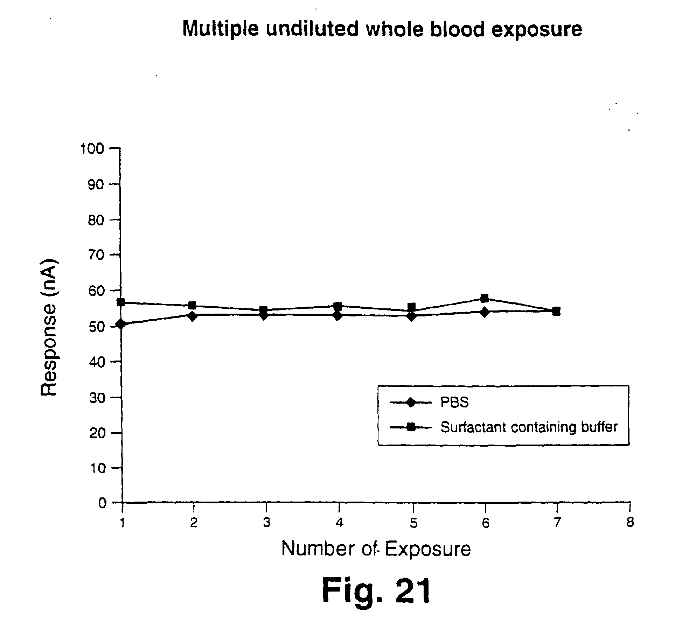

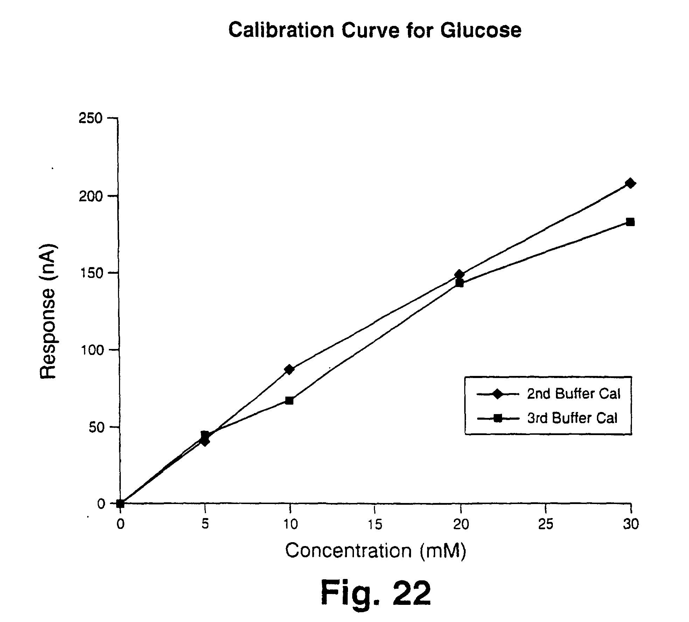

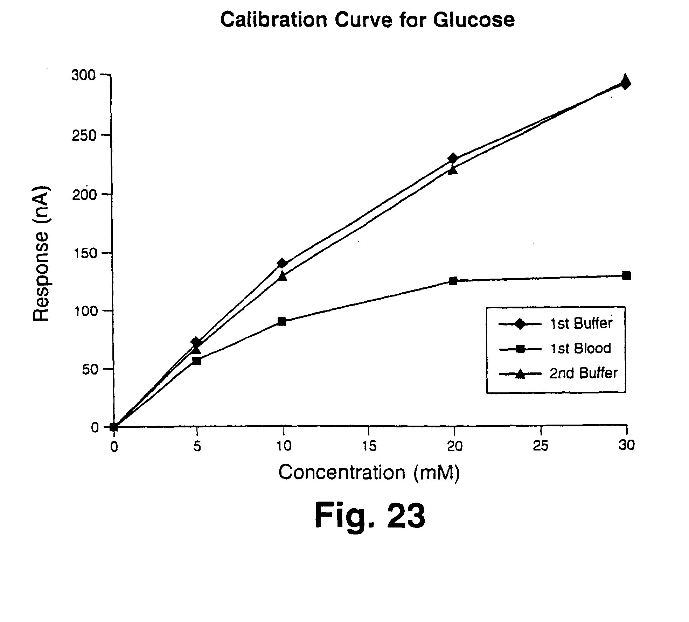

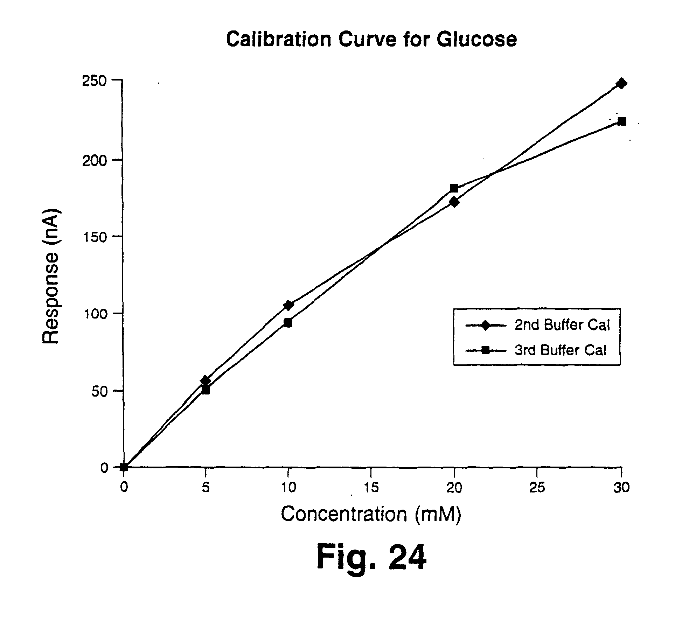

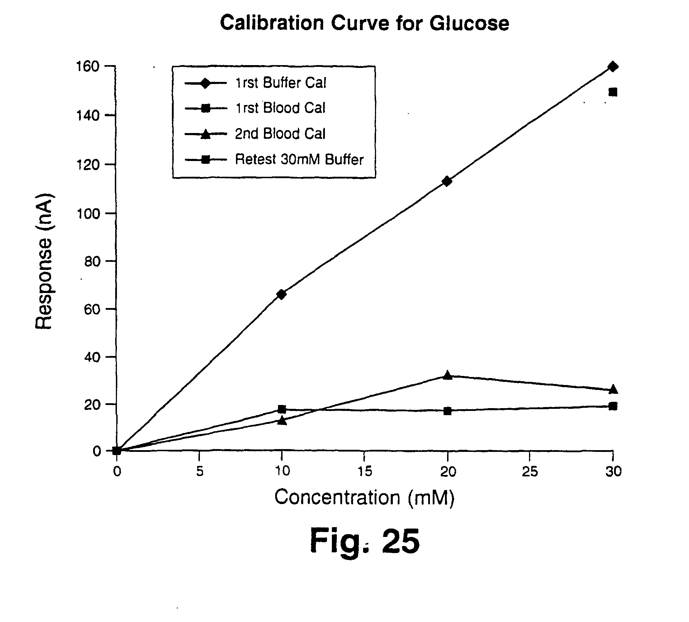

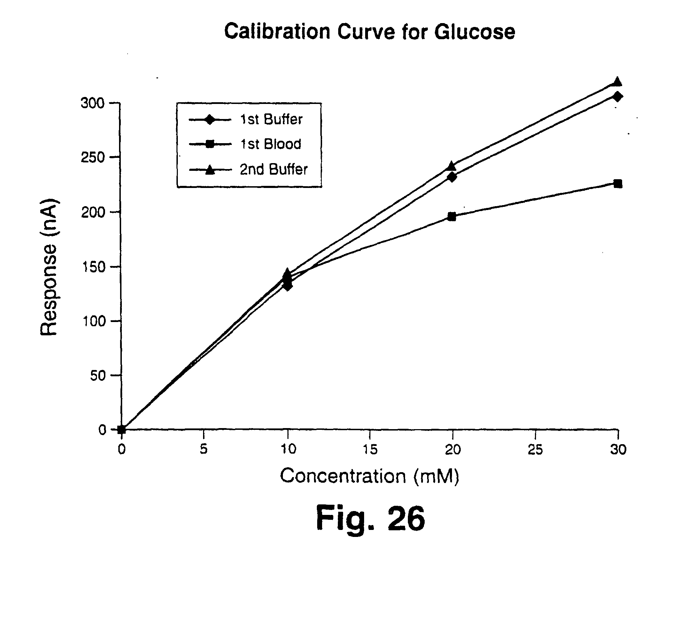

Figures 11 to 26 are graphs of various test results for test strips suitable for use in the present invention having further alternative electrode formulations using another different conductive ink;

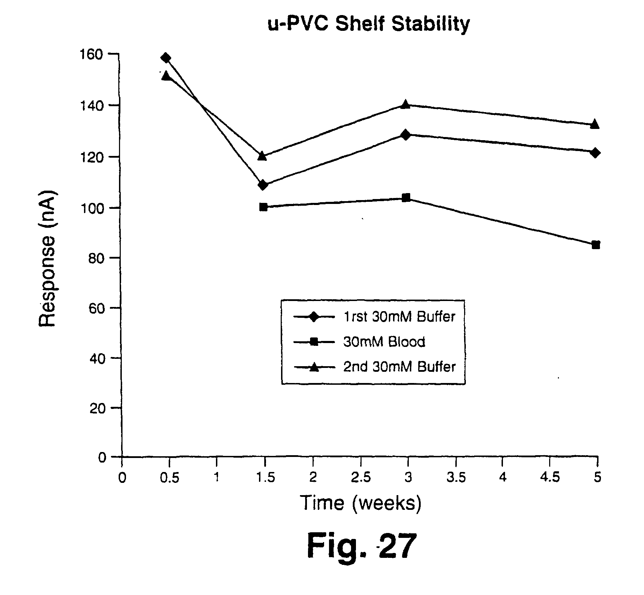

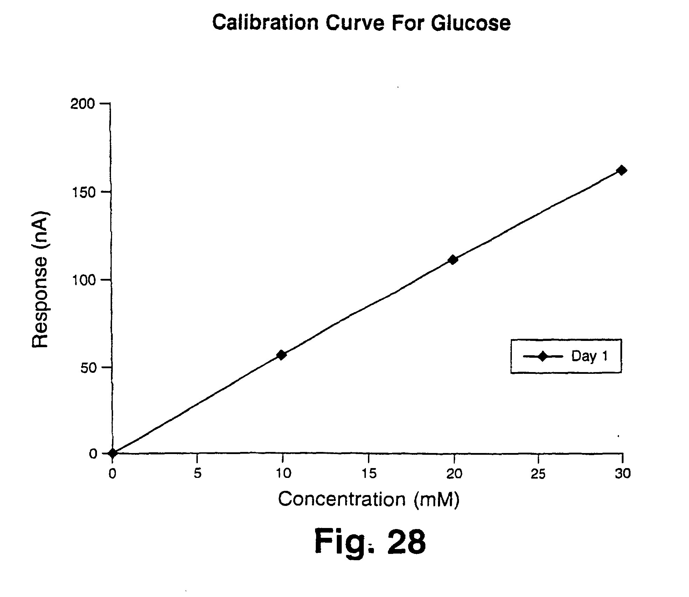

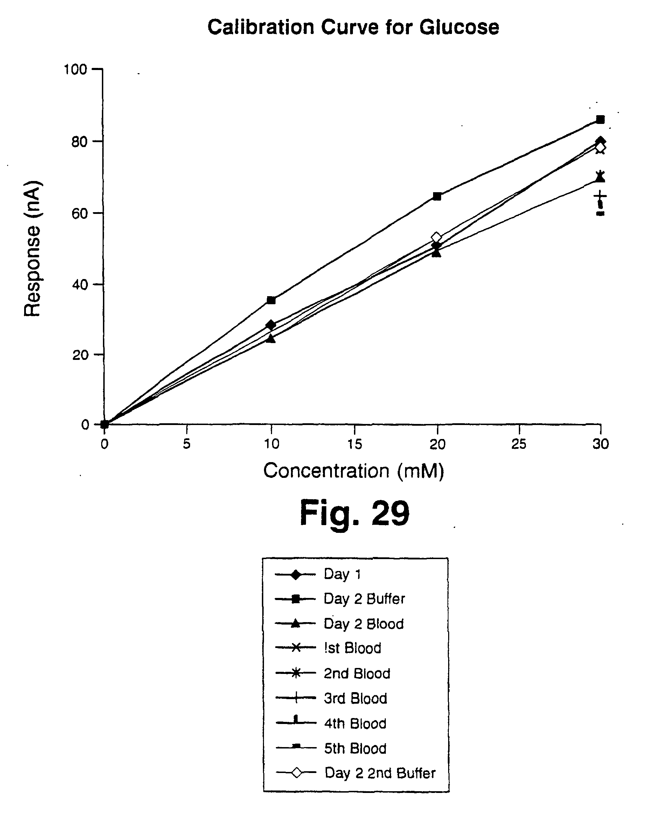

Figures 27 to 29 are graphs for various test results with PVC membranes and different electrode ink formulations;
   and
Figure 30 shows printed layers on a substrate for making a re-usable test member suitable for use in the present invention.

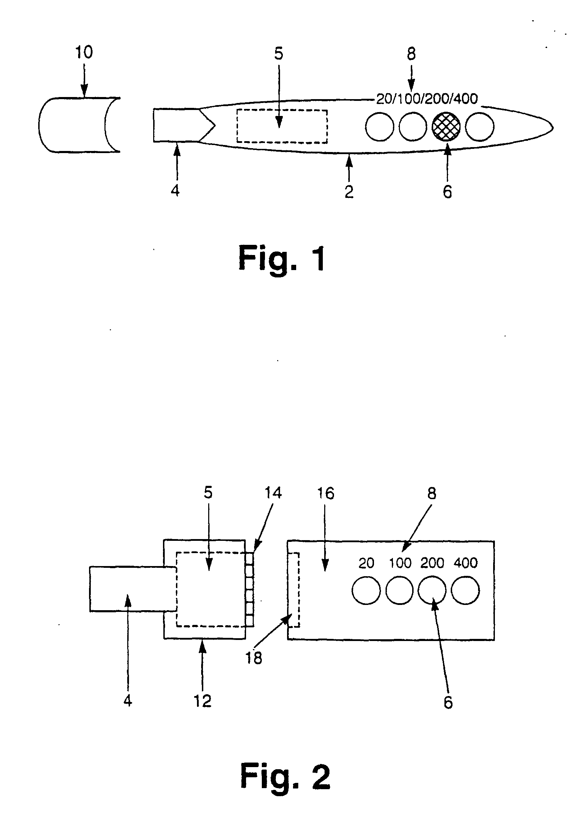

The test device 2 shown in Figure 1 comprises a meter 5 which is permanently connected to a re-usable test strip 4. The meter 5 has display means 6, comprising a bank of LEDs, and a printed scale 8 alongside the LEDs 8. Four LEDs are shown for the purposes of illustration, but any number could be used, for example 8 to 10 LEDs. The number of LEDs used would compare with the best visual check card on the market. One or more LCDs or other display devices could of course be used instead of LEDs. It is not necessary that a diabetic monitors his glucose level to the apparent accuracy of current glucose meters which are on the market. It is sufficient for the diabetic to know roughly what the level is (+/- 15 mg glucose per dl).

In addition to visual display means, the device may optionally be provided with means for audibly warning a user when a reading of analyte concentration is deemed too low or too high, outside a defined pre-programmed concentration range.

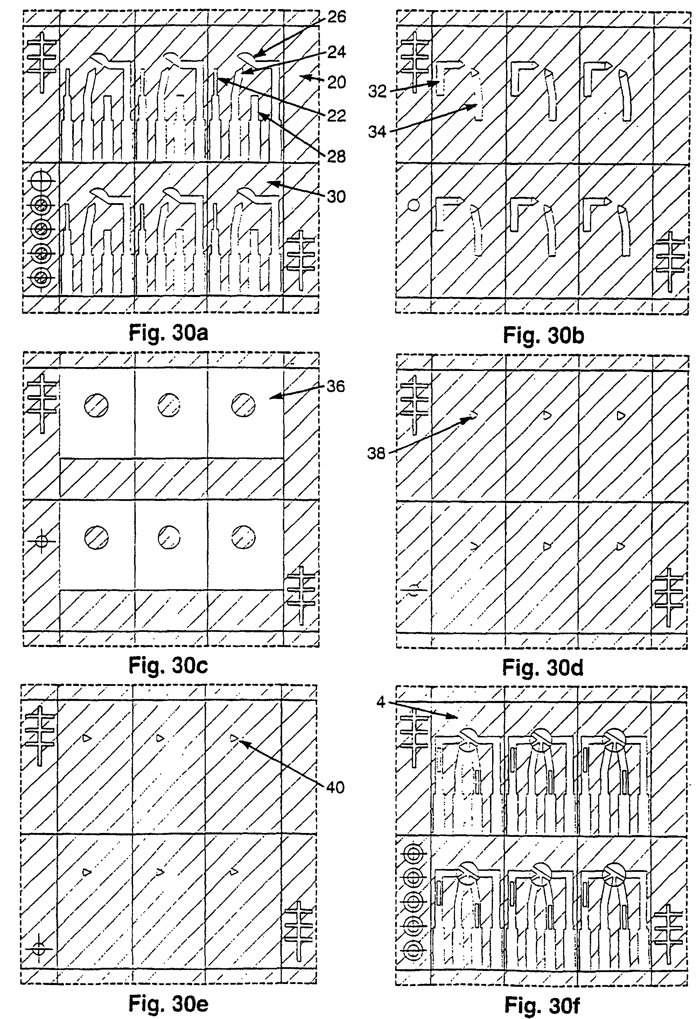

Referring now to Figure 30, the test strip 4 comprises a substrate 30 coated sequentially with a plurality of electrode and dielectric layers. Figure 30a shows a ceramic plate 20 which is marked into six test strip substrates 30. Other substrate materials may of course be used instead of a ceramic, for example polyester, polycarbonate, or PVC. Each substrate 30 is printed with conductive silver/silver chloride tracks, comprising: an active electrode 22, reference electrode 24, dummy electrode 28 and counter electrode 26. We have found that a ratio of silver to silver chloride in the range 60:40 to 95:5 gives best results. A particularly preferred ratio is about 90:10 Ag/AgCl.

After curing, the second layer is printed on, as shown in' Figure 30b. Base carbon tracks 32 and 34 (resistivity about 40 ohms per square) are printed for the active and dummy electrodes respectively. An insulating dielectric layer is then printed (Figure 30c), having a circular aperture where a fluid sample is to be received. This layer must be resistant to solvents, eg acetone, THF, and cyclohexanone. It must be stable in aqueous solutions, showing no cracking which would result in penetration of solution beneath the insulating layer and resulting in short-circuiting of the tracks. Figures 30d and 30e show respectively the working electrode 38 for the dummy electrode and the working electrode 40 for the active electrode. The complete electrode assembly is shown in Figure 30f, prior to (preferably) coating with a protective membrane and cutting into six test strips 4. After cutting, each test strip 4 is permanently attached to the meter 2 or a cartridge 12 as shown in Figure 2.

The working electrode 38 of the dummy electrode 28 comprises BSA adsorbed onto platinised carbon, and the working electrode 40 of the active electrode 22 comprises GOD adsorbed onto platinised carbon. In use, electrical potential from a battery carried by the meter 5 polarises the working electrodes 38,40 to between +200 mV and +600 mV relative to the reference electrode 24. We have found that a polarity of about +300 mV relative to the reference electrode 24 provides a suitable compromise between sensitivity and interference response. When blood containing glucose is applied to the test strip 4 a current is produced which is dependent on the concentration of glucose in the blood. Depending on the current passed, a particular LED 6 lights up, controlled by a microchip. The meter 5 is calibrated so that the printed scale 8 corresponds with a range of glucose concentrations in the applied blood. The user can therefore read off from the printed scale at the point adjacent to the lit LED 6, the concentration of blood glucose.

The test strip 4 has a protective cap 10 which covers the test strip 4 when secured on the housing of the meter 5 by means of a screw thread. As the user unscrews the cap 10 the test strip 4 is revealed and a counter is set. Once the test has been carried out, the cap 10 is replaced. In doing this, the strip 4 is cleaned so that its conductivity substantially returns to that of the test strip prior to testing. Cleaning may be effected by wiping with a solvent wipe contained within the cap 10, or by rinsing under a tap, or by abrading or removing one or more reagent layers from the test strip 4.

After a predetermined number of cycles of removal and replacement of the cap 10, a lock is actuated and the cap 10 becomes locked to the housing of the meter 5. The user then disposes of the test device and begins use of a new device.

The invention provides a test device with simple electronics, which can be individually calibrated in the factory. An LCD or an optical reader is not required, although the display means could include an LCD if preferred. The battery supplied with the device will outlast the envisaged life of the device, thereby eliminating the need for the user to replace batteries.

In an alternative embodiment of the invention shown in Figure 2, the test strip 4 is permanently connected to a cartridge 12. The cartridge 12 houses the meter 5 which includes electronic means for calibrating the test strip both in the factory and, if necessary, at other times during the life of the meter 5. The cartridge 12 carries an edge connector 14 at one end, for releasably connecting the cartridge to a socket 18 in a housing 16 for the display means 6. When the cartridge 12 is connected to the housing 16 the assembly functions essentially identically to the device of Figure 1. When the cartridge 12 comes to the end of its useful life, it can be disposed of, and a replacement cartridge can be fitted to the housing 16.

An alternative embodiment of the invention is to have the test strip contained within a pre-formed plastic casing. The plastic casing and strip will have a low void volume (<10ul). The plastic casing has an aperture at the furthest extremity of the strip, this allows blood to flow to the electrode surface by capillary forces. At the meter end of the plastic casing are two nipples, one is attached to a washing fluid reservoir and the other to a calibration solution reservoir. Both reservoirs are located within the meter, their total volume is 3ml. As the void volume of the system is less than 10ul, 30ul of washing solution is required after each test. This allows easy washing and calibration of the system with minimum fuss to the patient.

The preferred use of a membrane over the electrode improves stability of the test member over time, and permits the use of small (about 2 µl) samples. However, the membrane also reduces the sensitivity of the system, so it is desirable to have as highly sensitive a base electrode as possible. In particular it is desirable to increase conductivity of the electrode tracks, and to arrive at a working electrode formulation which permits a suitable shelf life.

A preferred membrane material is PVC, notably low molecular weight PVC, which linearises the response of the electrode and provides protection against the fouling' effects of dried blood contact on the electrodes by, for example, clot formation, adsorption of cells and proteins, and dried blood. It is particularly preferred that the membrane contains a surfactant to reduce fouling effects of dried blood.

Various electrode compositions and membranes were evaluated for use in the invention, and experimental results for these systems are discussed below.

### EXPERIMENTAL RESULTS

An electrode with relatively high sensitivity to glucose was prepared by first adsorbing BSA or GOD onto 10% platinised activated carbon, followed by production of the working inks with the BSA or GOD - Pt/carbon, and then screen printing of the various layers onto a ceramic substrate.

Preferred resins for making the inks are polyesters and acrylic materials. A polyester resin which is particularly preferred for this application is Metech 8101.

References to "low molecular weight PVC" or the like refer to a PVC having a molecular weight of about 100,000. Specifically, PVC product code Norvinyl S6036 from Hydro Polymers Limited, Newton Aycliffe, County Durham, DL5 6EA UK, was used.

### Preparation of platinised carbon ink for the dummy and active electrode

### 1) Adsorption of protein to 10% platinised carbon

### Materials

Glucose oxidase (GOD), Type GO3A from Biozyme.
Bovine serum albumin (BSA), Fraction V from Pentex (Miles Inc).
10% platinum loaded Vulcan XC72R activated carbon commercially available from E-Tek Inc prepared from the deposition of colloidal platinum (particle sise 1.5 to 2.5nm) onto the surface of activated carbon particles (nominal sise 30nm) by oxidative decomposition of complex platinum sulfite acid (II) using hydrogen peroxide.
Phosphate buffered saline pH 7.4 (PBS), consisting of 10mM sodium phosphate, 137mM NaCl, 2.7 mM KCl.
Whatman no.1 qualitative filter paper.

### Method

### (a) Preparation of BSA-Pt/carbon

In a 250ml glass bottle, 6.4g of BSA was dissolved in 80ml of PBS and 20g of 10%Pt/XC72R gradually added with constant stirring. The bottle was then placed on a roller mixer and allow to incubate overnight at room temperature. A Buchner funnel was prepared with two pieces of filter paper. The mixture was poured into the funnel and the carbon washed three times with approximately 100ml of PBS. The vacuum was allowed to pull through the cake of carbon for about five minutes to extract as much liquid as possible and to aid in the drying of the carbon. The cake of carbon was carefully scraped out into a plastic container and broken up with a spatula. The carbon was then placed in an oven at 30°C overnight to dry. The carbon was stored in a refrigerator at 4°C if not immediately used.

### (b) Preparation of GOD-Pt/carbon

This was prepared using exactly the same method as (a), with GOD in place of BSA.

### 2) Preparation of inks

These inks were prepared by Gwent Electronic Materials using the above BSA and GOD Pt/carbons..

### Materials

BSA/Pt-carbon or GOD/Pt-carbon prepared in (1) above Metech 8101 polyester type resin as the polymer binder Terpineol BP from RC Treatt, an oil to act as a flow agent Butyl cellosolve acetate, a solvent to adjust the ink viscosity

The formulation of the ink consisted of:

| | |
|---|---|
| Metech 8101 resin | 54.05% |
| BSA or GOD Pt-carbon | 27.09% |
| Butyl cellosolve acetate | 12.57% |
| Terpineol BP | 6.29% |

The liquid components were blended together and the carbon added. After initial handmixing, the mixture was passed several times through a triple roll mill to produce a smooth homogenous carbon ink suitable for screen printing. The ink was left to stand for several hours, preferably overnight, to allow thorough wetting of the carbon particles with resin before use.

The product code numbers assigned by Gwent Electronic Materials was C80707R2 ("R2") and C80707R3 ("R3") for the BSA and GOD ink respectively.

### 3) Screen printing of electrodes

### Materials

Screens used were obtained from DEK Precision Screen Division and were of 325 mesh and 230 mesh depending on the ink printed. 325 mesh screens were used for the Ag/AgCl and dielectric ink. 230 mesh was used for printing of the carbon inks. All meshes were of 13µm emulsion thickness and were of 45° orientation.
Ag/AgCl (90:10 ratio) ink from Gwent Electronic Materials Ltd, product code C80205D1
Conductive carbon (40Ω/□) from Gwent Electronic Materials Ltd, product code C80130D1
Insulating dielectric from Gwent Electronic Materials Ltd, product code D80601D6
Platinised carbon ink for dummy (BSA) electrode - as prepared above (C80707R2)
Platinised carbon ink for active (GOD) electrode - as prepared above (C80707R3)
Prescored ceramic substrates from the Laser Cutting Company

The electrodes were fabricated by screen printing each layer on top of each other using a DEK1200 screen printing machine.

### 4 Electrochemical measurements

The electrodes were polarised to +300mV, the working potential, with reference to the printed silver/silver chloride reference electrode. The current response on application of sample was measured at either fixed time intervals, eg 30 or 60 seconds. In the case of membrane covered electrodes, the electrodes were preconditioned by hydrating and maintaining at +300mV for 30 minutes. The use of a bipotentiostat (from Cambridge Life Sciences plc) enables the simultaneous polarisation and measurement of both the dummy and active electrodes.

The dummy electrode, containing an inert non-enzymic protein, eg bovine serum albumin, will respond non-specifically to any background electroactive species, eg ascorbate, urate, acetaminophen (Paracetamol). The active electrode which contains glucose oxidase, will in addition to background electroactive species, respond specifically to the presence of glucose in the sample. The subtraction of these two responses gives the analytical response specifically due to the presence of glucose in the sample.

### Results

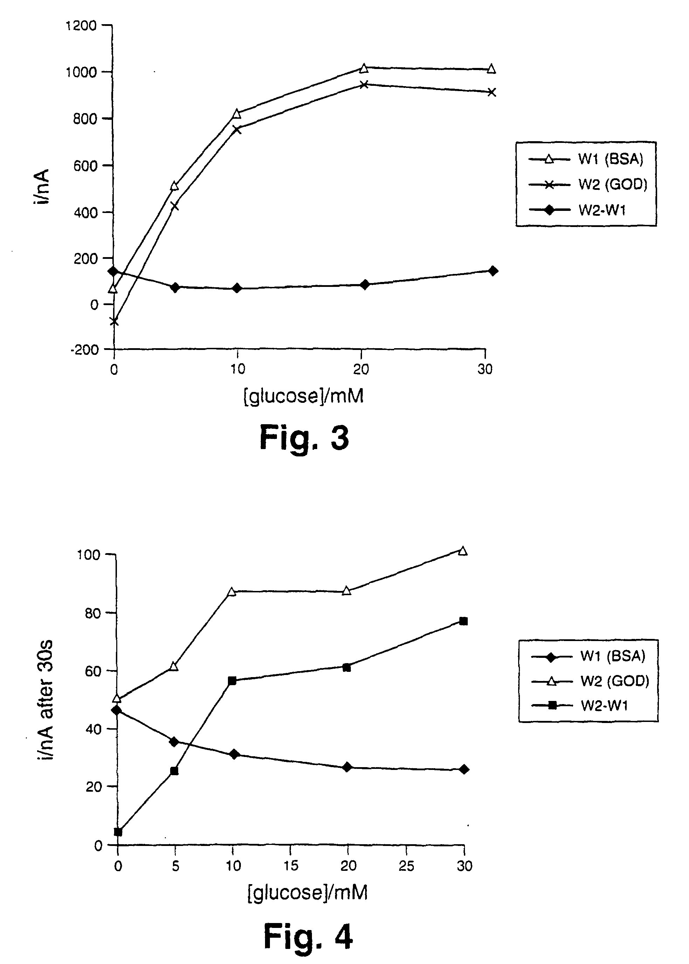

Figure 3 shows the graph for buffer glucose calibration of the bare electrode, average response at 30 s. Figure 4 shows the graph for buffer glucose calibration for the electrode coated with a PVC membrane coated from a 7% cyclohexanone solution by screen printing. Corrected current values in excess of 50 nA were obtained for glucose concentrations over 10 mM.

### PVC Membrane - R6/R7

Further electrodes were prepared in a similar method to above, involving preparation of the BSA and GOD platinised carbons, production of the working inks, and screen printing of the various layers to form the complete glucose biosensor.

### 1) Adsorption of protein to 5% platinised carbon

### Method

### Materials

Glucose oxidase (GOD), Type GO3A from Biozyme.
Bovine serum albumin (BSA), Fraction V from Pentex (Miles Inc).
5% platinum loaded Vulcan XC72R activated carbon comercially available from E-Tek Inc prepared from the deposition of colloidal platinum (particle sise 1.5 to 2.5nm) onto the surface of activated carbon particles (nominal sise 30nm) by oxidative decomposition of complex platinum sulfite acid (II) using hydrogen peroxide.
Phosphate buffered saline pH 7.4 (PBS), consisting of 10mM sodium phosphate, 137mM NaCl, 2.7 mM KCl.
Whatman no.1 qualitative filter paper.

### (a) Preparation of BSA-Pt/carbon

In a 250ml glass bottle, 6.4g of BSA was dissolved in 160ml of PBS and 40g of 5%Pt/XC72R activated carbon added with constant stirring. The bottle was then placed on a roller mixer and allowed to incubate overnight at room temperature. A Buchner funnel was prepared with two pieces of the filter paper. The mixture was poured into the funnel and the carbon washed three times with approximately 100ml of PBS. The vacuum was allowed to pull through the cake of carbon for about a further five minutes to extract as much liquid as possible and to aid in drying. The cake of carbon was carefully scraped out into a plastic container and the lumps broken up with a spatula. The carbon was placed in an oven at 30°C overnight to dry. The carbon was then stored in a refrigerator at 4°C if not immediately used.

### (b) Preparation of GOD-Pt/carbon

This was prepared using exactly the same method as (a), with 5.7g of GOD dissolved in 144ml of buffer then adding 36g of 5%Pt/XC72R activated carbon powder.

### 2) Preparation of working inks

### Materials

BSA/Pt-carbon or GOD/Pt-carbon prepared in (1a and 1b) above
Metech 8101 polyester type resin as the polymer binder Terpineol BP from RC Treatt, an oil to act as a flow agent Tergitol NP-10 from Surfachem, a surfactant to act as a wetting agent
Butyl cellosolve acetate, a solvent to adjust the ink viscosity
Cellosolve acetate, a solvent to adjust the ink viscosity

### Method

These inks were prepared by Gwent Electronic Materials using the above BSA and GOD Pt/carbons using the following formulations:

| **BSA ink for the dummy electrode** | |
|---|---|
| Metech 8101 resin | 43.11% |
| BSA Pt-carbon | 21.55% |
| Butyl cellosolve acetate | 17.30% |
| Terpineol BP | 11.84% |
| Tergitol NP10 | 3.30% |
| Cellosolve acetate | 2.90% |

This was assigned the product code C80506R6 ("R6") by Gwent Electronic Materials

| **GOD ink for the active electrode** | |
|---|---|
| Metech 8101 resin | 47.16% |
| BSA Pt-carbon | 23.58% |
| Butyl cellosolve acetate | 11.72% |
| Terpineol BP | 11.15% |
| Tergitol NP10 | 3.61% |
| Cellosolve acetate | 2.78% |

This was assigned the product code C80506R7 ("R7") by Gwent Electronic Materials. The liquid components were blended together and the carbon added. After initial handmixing, the mixture was passed several times through a triple roll mill to produce a smooth homogenous carbon ink suitable for screen printing.
The ink was left to stand for several hours, preferably overnight, to allow thorough wetting of the carbon particles with resin before use.

### 3) Screen printing of electrodes

### Materials

Screens used were obtained from DEK Precision Screen Division and were of 325 mesh and 230 mesh depending on the ink printed. 325 mesh screens were used for the Ag/AgCl and dielectric ink. 230 mesh was used for printing of the carbon inks. All meshes were of 13µm emulsion thickness and were of 45° orientation.
Ag/AgCl (90:10 ratio) ink from Gwent Electronic Materials Ltd, product code C80205D1
Conductive carbon (40Ω/□) from Gwent Electronic Materials Ltd, product code C30130D1
Insulating dielectric from Gwent Electronic Materials Ltd, product code D80601D6
Platinised carbon ink for dummy (BSA) electrode - as prepared above (C80506R6)
Platinised carbon ink for active (GOD) electrode - as prepared above (C80506R7)
Prescored ceramic substrates from the Laser Cutting Company

The electrodes were fabricated by screen printing each layer on top of each other using a DEK1200 screen printing machine.

### 4) Deposition of biocompatible PVC membrane by spincoating and screen printing

### Materials

Screen printed electrodes from 3 above.
Polyvinylchloride (PVC), low molecular weight, from Hydro Polymers Ltd
Pluronic F68 (P-7061) from Sigma
Tetrahydrofuran (THF), from Ultrafine Ltd
Cyclohexanone from Fisher Scientific UK

### 4.1 Method for spin coating of electrodes

### (a) Preparation of spin coating PVC mixtures

Two types of PVC membrane have been used: surfactant modified and unmodified PVC membrane.
**(i):** 0.70g of PVC was dissolved in 10ml THF by stirring. This gave a concentration of 7%w/v of unmodified PVC membrane solution.
**(ii):** 0.80g of PVC and 0.1g of Pluronic F68 were dissolved in 10ml THF with constant stirring. This produced an 8%w/v PVC/1%w/v Pluronic F68 membrane solution.

1. Cover the contact legs with a non-adhesive paper.
2. Place two electrodes at the centre of the spin disk (side by side and working area near centre of disk).
3. Tape the electrodes down tightly.
4. Mount the spin disk on the spin coat machine.
5. Switch on the machine.
6. Adjust the speed to 1000rpm.
7. Add 1ml membrane solution at the centre of the disk at 5-6cm height.
8. After 1 minute, remove the fabricated sensors from the disk and leave in fume cupboard overnight.
9. Dry the fabricated sensors in Vacuum oven evacuated by 0.85 bar at room temperature for 1 hour.

### 4.2 Method for screen printing PVC mixtures

### Preparation of screen printing PVC mixture

1.214g of PVC and 0.143g of Pluronic F68 were dissolved in 10ml cyclohexanone with constant stirring.

A screen with 325 mesh, 13µm emulsion thickness and 45° orientation was used to print the polymer.

### 5 Haematocrit study

Venous blood was prepared to give haematocrits (% red blood cells in total blood) in the range 15 - 60%. The response of the electrodes was compared with a Yellow Springs Instrument (YSI) as the reference method.

### 6 Electrochemical measurements

The electrodes were polarised to +300mV, the working potential, with reference to the printed silver/silver chloride reference electrode. The current response on application of sample was measured at either fixed time intervals, eg 30 or 60 seconds. In the case of membrane covered electrodes, the electrodes were preconditioned by hydrating and maintaining at +300mV for 30 minutes. The use of a bipotentiostat (from Cambridge Life Sciences plc) enables the simultaneous polarisation and measurement of both the dummy and active electrodes.

The dummy electrode, containing an inert non-enzymic protein, eg bovine serum albumin, will respond non-specifically to any background electroactive species, eg ascorbate, urate, acetaminophen (Paracetamol). The active electrode which contains glucose oxidase, will in addition to background electroactive species, respond specifically to the presence of glucose in the sample. The subtraction of these two responses gives the analytical response specifically due to the presence of glucose in the sample.

In the tables of results below, membranes are denoted by their weight % in the solution from which they were applied. The electrode used for the graphs of Table 1 below was a 90:10 mixture of R6 & R7.

**Table 1**

| **Results - R6/R7 (90:10)** | |
|---|---|
| Fig. 5 | Bare electrode response to glucose calibration. Room temp. 300 mV; response time: fixed 60 s; PBS buffer; stability: 5.2% drop at day 2; 29% drop at day 15; 31% drop at day 20; 45% drop at day 34. |
| Fig. 6 | Electrode with spin coated 8%PVC+1%Pluronic F68, 900 rpm. PBS buffer; Room temperature; 300 mV; Response time: fixed 60 s; recovery time 3-4 minutes. |
| Fig. 7 | Electrode with screen printed 12.14%PVC+1.43%Pluronic F68 buffer glucose and blood glucose calibration curves. Room temperature; PBS buffer; 300 mV, 4 electrode polarisation; Response time: fixed 60 s; Condition: 30 minutes hydration time. |
| Fig. 8 | Dependence on electrode response to haematocrit compared to a reference method using a Yellow Springs Instrument. Room temp. Membrane: 8%PVC & 1% Pluronic F68; PBS buffer; 300 mV. |
| Fig. 9 | In-use stability of membrane covered electrode (spin coated 8%low mwt PVC+1%Pluronic F68) at room temperature. Sensor evaluation: sensor polarised for 30 min. in Use Stability at room temp. PBS buffer; signal processing: 60 s; signal reduction: 19% week 2, 21% week 5. |
| Fig. 10 | In-use stability of membrane covered electrode (spin coated 7% low mwt PVC) at room temperature. Sensor polarised for 30 min. in Use Stability at room temp; PBS buffer; +300 mV; signal processing: 60 s; signal reduction: 21% week 2; 27% week 5. |
| Fig. 11 | In-use stability of membrane covered electrode (spin coated 8%PVC+1%Pluronic F68) at 37°C; PBS buffer; +300 mV; signal processing: 60 s; signal reduction: 20% on day 3;27% on day 11;54% on week 5 |
| Fig. 12 | In-use stability of membrane covered electrode (spin coated 7% low mwt PVC) at 37°C; PBS buffer; +300 mV; sensor polarised for 30 minutes in Use Stability at 37°C; signal processing: 60 s; signal reduction: 6% on day 3; 31% on day 11; 43% on week 5. |
| Fig. 13 | Dependence of response to sample volume. Membrane: 8% low mwt PVC & !% Pluronic F68; sensor polarised for 30 minutes; sensor tested with 30 mM blood glucose; PBS buffer; room temp. +300 mV; signal processing 30 s & 60 s. |

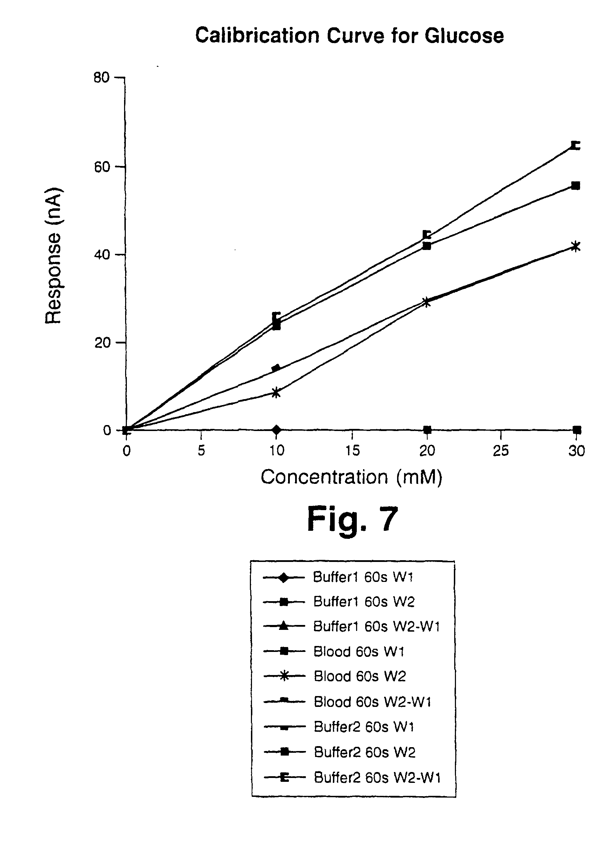

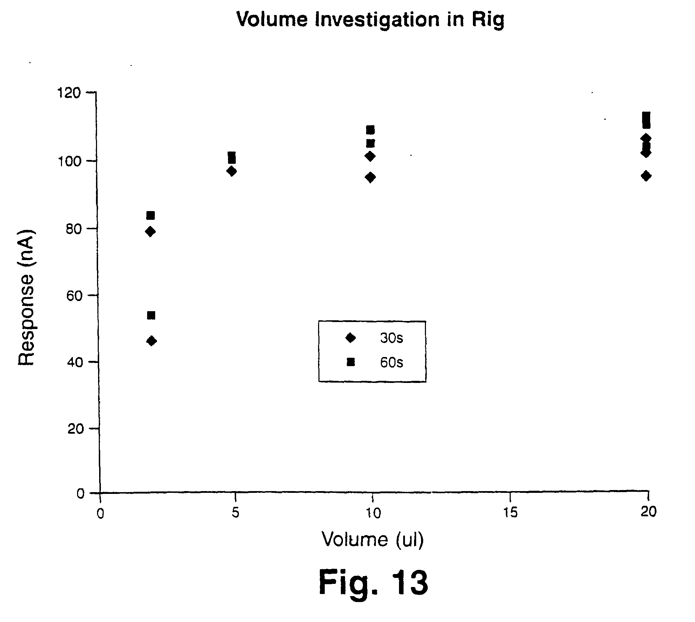

In the results shown in Figure 7, the dummy electrode values (W1) are virtually zero. The volume investigation graphed in Figure 13 shows that for a typical small sample volume of 5 µl, relatively high response currents (about 100 nA) can be obtained using a membrane system. No attempt was made to pre-condition the electrodes prior to undegoing in-use stability trials. We believe there is an initial 10-20% drop in response at an early time point in the strip's life, and that it is substantially stable thereafter.

### Electrode with results for PVC, CA and PU membranes (R3/R5 results)

Further experimental work was carried out using another different electrode system and various membranes.

### Materials

Glucose oxidase (GOD), Type GO3A from Biozyme.
Bovine serum albumin (BSA), Fraction V from Pentex (Miles Inc).
5% platinum loaded Vulcan XC72R activated carbon comercially available from E-Tek Inc prepared from the deposition of colloidal platinum (particle sise 1.5 to 2.5nm) onto the surface of activated carbon particles (nominal sise 30nm) by oxidative decomposition of complex platinum sulfite acid (II) using hydrogen peroxide.
Phosphate buffered saline pH 7.4 (PBS), consisting of 10mM sodium phosphate, 137mM NaCl, 2.7 mM KCl.
Whatman no.1 qualitative filter paper.

### 1 Preparation of working carbon inks

### (a) Preparation of BSA-Pt/carbon

In a 250ml glass bottle, 6.4g of BSA was dissolved in 160ml of PBS and 40g of 5%Pt/XC72R activated carbon added with constant stirring. The bottle was then placed on a roller mixer and allowed to incubate overnight at room temperature. A Buchner funnel was prepared with two pieces of the filter paper. The mixture was poured into the funnel and the carbon washed three times with approximately 100ml of PBS. The vacuum was allowed to pull through the cake of carbon for about a further five minutes to extract as much liquid as possible and to aid in drying. The cake of carbon was carefully scraped out into a plastic container and the lumps broken up with a spatula. The carbon was placed in an oven at 30°C overnight to dry. The carbon was then stored in a refrigerator at 4°C if not immediately used.

### (b) Preparation of GOD-Pt/carbon

This was prepared using exactly the same method as (a), with 5.7g of GOD dissolved in 144ml of buffer then adding 36g of 5%Pt/XC72R activated carbon powder.

### Materials

BSA/Pt-carbon or GOD/Pt-carbon prepared in (1a and 1b) above
Metech 8101 polyester type resin as the polymer binder Tergitol NP-10 from Surfachem, a surfactant to act as a wetting agent
Butyl cellosolve acetate, a solvent to adjust the ink viscosity

These inks were prepared by Gwent Electronic Materials using the above BSA and GOD Pt/carbons using the following formulations:

| **BSA ink for the dummy electrode** | |
|---|---|
| Metech 8101 resin | 50.56% |
| BSA Pt-carbon | 25.28% |
| Butyl cellosolve acetate | 20.80% |
| Tergitol NP10 | 3.36% |

This was assigned the product code C80319R3 ("R3") by Gwent Electronic Materials.

### 2) Screen printing of electrodes

### Materials

Screens used were obtained from DEK Precision Screen Division and were of 325 mesh and 230 mesh depending on the ink printed. 325 mesh screens were used for the Ag/AgCl and dielectric ink. 230 mesh was used for printing of the carbon inks. All meshes were of 13µm emulsion thickness and were of 45° orientation.
Ag/AgCl (90:10 ratio) ink from Gwent Electronic Materials Ltd, product code C80205D1
Conductive carbon (40Ω/□) from Gwent Electronic Materials Ltd, product code C80130D1
Insulating dielectric from Gwent Electronic Materials Ltd, product code D80601D6
Platinised carbon ink for dummy (BSA) electrode - as prepared above (C80506R6)
Platinised carbon ink for active (GOD) electrode - as prepared above (C80506R7)
Prescored ceramic substrates from the Laser Cutting Company

The electrodes were fabricated by screen printing each layer on top of each other using a DEK1200 screen printing machine.

| **GOD ink for the active electrode** | |
|---|---|
| Metech 8101 resin | 54.79% |
| BSA Pt-carbon | 27.40% |
| Butyl cellosolve acetate | 13.62% |
| Tergitol NP10 | 4.19% |

This was assigned the product code C80319R5 ("R5") by Gwent Electronic Materials.

The liquid components were blended together and the carbon added. After initial handmixing, the mixture was passed several times through a triple roll mill to produce a smooth homogenous carbon ink suitable for screen printing. The ink was left to stand for several hours, preferably overnight, to allow thorough wetting of the carbon particles with resin before use.

### 3 Method for spin coating of electrodes

### Materials

Polyvinylchloride (PVC), low molecular weight, from Hydro Polymers Ltd
Pluronic F68 (P-7061) from Sigma
Cellulose acetate (CA), acetyl content ⁻40% (C3782) from Sigma
Polyurethane (PU), Trixene (SC7602), from Baxenden Chemicals
Acetone
Tetrahydrofuran (THF), from Ultrafine Ltd

### (a) Preparation of spin coating polymer mixtures

Surfactant modified and unmodified polymer membranes preparation depended on the type of polymer used.

PVC was dissolved in THF to the required concentration. For example, a 7% solution of unmodified PVC will contain 0.7g of PVC dissolved in 10ml THF. An 8% solution of PVC modified with 1% Pluronic F68 will contain 0.8g of PVC and 0.1g of Pluronic F68 dissolved in 10ml of THF.

Cellulose acetate was dissolved in acetone to the required concentration. For example, a 2% solution of cellulose acetate will consist of dissolving 0.2g of cellulose acetate in 10ml of acetone. A modified solution can be prepared by adding additional modifiers. For example, to this solution, 0.05g of Pluronic F68 can be added to produce a solution containing 2% cellulose acetate and 0.5% Pluronic F68.

Polyurethane was similarly prepared. A 4% solution of polyurethane will consist of dissolving 0.4ml of polyurethane (Trixene) into 10ml of THF. To this Pluronic F68 can be added to produce a modified membrane solution. For example, to this solution, 2% Pluronic F68 would involve adding 0.2g to the solution.

### (b) Spin coating procedure

1. Cover the contact legs with a non-adhesive paper.
2. Place two electrodes at the centre of the spin disk (side by side and working area near centre of disk).
3. Tape the electrodes down tightly.
4. Mount the spin disk on the spin coat machine.
5. Switch on the machine.
6. Adjust the speed to 1000rpm.
7. Add 1ml membrane solution at the centre of the disk at 5-6cm height.
8. After 1 minute, remove the fabricated sensors from the disk and leave in fume cupboard overnight.
9. Dry the fabricated sensors in Vacuum oven evacuated by 0.85 bar at room temperature for 1 hour.

### 3 Electrochemical measurements

The electrodes were polarised to +300mV, the working potential, with reference to the printed silver/silver chloride reference electrode. The current response on application of sample was measured at either fixed time intervals, eg 30 or 60 seconds. In the case of membrane covered electrodes, the electrodes were preconditioned by hydrating and maintaining at +300mV for 30 minutes. The use of a bipotentiostat (from Cambridge Life Sciences plc) enables the simultaneous polarisation and measurement of both the dummy and active electrodes.

The dummy electrode, containing an inert non-enzymic protein, eg bovine serum albumin, will respond non-specifically to any background electroactive species, eg ascorbate, urate, acetaminophen (Paracetamol). The active electrode which contains glucose oxidase, will in addition to background electroactive species, respond specifically to the presence of glucose in the sample. The subtraction of these two responses gives the analytical response specifically due to the presence of glucose in the sample.

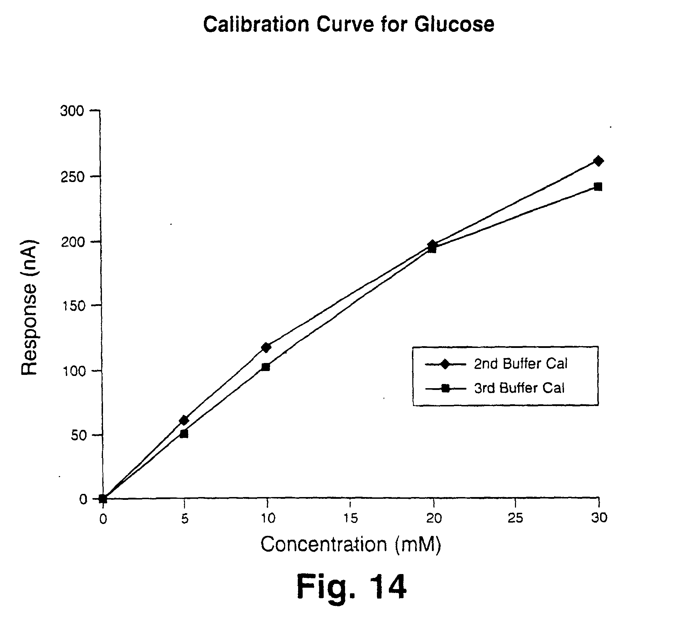

Figure 14 shows the calibration curve for an R3/R5 (90:10) glucose electrode with a membrane of low mwt PVC (0.1% w/v) from Hydropolymers. Sensor evaluation: Sensor used twice previously; 3^{rd} buffer calibration is after dried blood exposure overnight, followed by washing with a PBS buffer; room temperature; +300 mV; response time fixed 60 s. The curves are very similar, showing no significant fouling effect from the exposure to dried blood. A user could therefore wash the reusable test strip at any convenient time after taking a reading.

Results for R3/R5 (90:10) glucose electrodes with cellulose acetate and PVC membranes are given in Table 2 below. In each case the voltage was 300 mV. The buffer was PBS except where otherwise indicated.

**Table 2**

| **Results for R3/R5 electrodes (90:10) with no membrane and covered with cellulose acetate and PVC membranes** | |
|---|---|
| Fig. 15 | Bare R3/R5 electrode response; (no membrane); response time: fixed 60 s; Recovery Time: 4-5 min. day 1; 3-5 min. day 2 and day 3. |
| Fig. 16 | Electrode with 4% unmodified cellulose acetate membrane, buffer glucose response. Room temp. Response time: fixed 60 s; recovery time 4 min day 1. |
| Fig. 17 | Electrode with 4% cellulose acetate membrane modified with 0.5% Pluronic F68, buffer glucose response. Room temp. Response time: fixed 60 s; Recovery time: 2-5 min day 1. |
| Fig. 18 | Electrode with 0.1% low mwt PVC (Hydropolymers) allowed to air dry overnight and vacuum dried before testing; buffer glucose response. Room temp. |
| Fig. 19 | Bare electrode response to buffer and blood glucose response. Room temp; signal processing; 60 s time point. |
| Fig. 20 | Electrode with 4% cellulose acetate membrane, buffer and blood glucose response; buffer PBS and surfactant containing buffer; room temp. response time: fixed 60 s. |
| Fig. 21 | Electrode with 6% cellulose acetate + 1% Pluronic F68 membrane, multiple blood exposures and effect of washing solution; buffer: PBS + surfactant containing buffer; room temp. Response time: fixed 60 s. |
| Fig. 22 | Electrode with 0.1% cellulose acetate membrane (Sigma), showing no effect of blood fouling between buffer glucose calibration curves; room temp; Response time: fixed 60 s |

**Table 3**

| **Results for R3/R5 (90:10) electrodes covered with polyurethane (PU) membranes** | |
|---|---|
| Fig. 23 | Electrode with 4% PU/0.5% w/v Pluronic F68, showing buffer and blood calibrations not linear; PBS buffer; room temp. Response time fixed 60 s. 300 mV. |
| Fig. 24 | Electrode with 4% v/v PU (Trixene)/2% w/v Pluronic F68, showing no fouling effect from dried blood with membrane containing the Pluronic F68 modifier; sensor used twice previously; 3^{rd} buffer cal. is after dried blood exposure overnight, followed by cleaning in PBS for 1 hour; room temp. Response time: fixed 60 s; 300 mV, |
| Fig. 25 | Electrode with 15% PU (Trixene) membrane showing poor calibration, suggesting fouling effect of blood without Pluronic F68 modifier; Sensor evaluation: sensor polarised for 30 minutes; multiple exposures at 30 mM blood glucose also investigated; PBS buffer; +300 mV; signal processing: 60 s time point. |
| Fig. 26 | Electrode with 15% v/v PU + 1% Pluronic F68 showing improved resistance to blood fouling with inclusion of Pluronic F68 modifier; 300 mV; Recovery time 3-4 min.; Response time: fixed 60 s; Condition: 0.5 hr hydration, polarised. |

**Table 4**

| **Results for various electrode covered with polyvinylchloride (PVC) membranes** | |
|---|---|
| Fig. 27 | Shelf storage stability with 5 weeks data on R6/R7 (90:10) electrode with 7% unmodified low mwt PVC membrane. Storage in a dark and dry place at ambient temperature; sensor polarised for 30 minutes; signal processing: 60 s; signal reduction: 18% on week 3; 23% on week 5. |
| Fig. 28 | R3/R5 (60:40) Electrode with 8%PVC+1%Pluronic F68 membrane. Buffer glucose calibration; PBS buffer; room temp. Response Time: fixed 60 s; Recovery Time: 4 min. Condition: 30 minutes hrydration, polarised. |
| Fig. 29 | R3/R5 (90:10) Electrode with 8% w/v PVC+1% w/v Pluronic F68 membrane. Buffer and blood glucose calibration; room temp; PBS buffer; Response time: fixed 60 s. Recovery time: 4 min. Condition: 30 minutes hydration, polarised. |

## Claims

1. A device (2) for point of care testing of analyte concentration in a fluid applied thereto, the device comprising a test member (4) provided with sensing means adapted to produce an electrical signal in response to the concentration of analyte in an applied fluid, and a meter (5) comprising electronics means for producing an output which is dependent on the electrical signal from the test member (4), the meter (5) being provided with display means (6) for displaying the signal output; **characterised in that** the test member (4) is re-usable and permanently secured in relation to at least a part of the meter (5).

2. A device as claimed in Claim 1, wherein the sensing means comprises one or more reagents which react with the analyte to produce at least one electroactive species, and an electrical circuit which passes a current the magnitude of which varies according to the concentration of applied analyte.

3. A device as claimed in Claim 1 or Claim 2, wherein the test member (4) is provided with a cap (10) which covers the area to which fluid is to be applied, the inside surface of the cap (10) being provided with means for wiping or washing the test member (4) when the cap is removed from and/or replaced on the test member.

4. A device as claimed in Claim 3, further including a counter which records each instance when the cap (10) is removed from and/or replaced on the test member (4).

5. A device as claimed in Claim 4, which further includes locking means for locking the cap (10) on the test member (4) and means for actuating the locking means when the counter records a predetermined number.

6. A device as claimed in any one of the preceding claims, wherein the display means (6) comprises a plurality of light emitting diodes, each of which corresponds to an adjacent printed scale (8).

7. A device as claimed in any one of the preceding claims, wherein the meter (5) includes electronic calibration means for calibrating the signal output from the meter (5) in response to the electrical signal from test member (4) when a known concentration of analyte is applied thereto.

8. A device as claimed in any one of the preceding claims, wherein at least some of the electronics means of the meter (5) is permanently secured in relation to the test member (4) and is housed in a cartridge (12) which is releasably secured to a housing (16) which carries the display means (6).

9. A device as claimed in Claim 8, wherein the electronics means housed in the cartridge (12) includes calibration means for calibrating the signal output from the meter (5) in response to the electrical signal from test member (4) when a known concentration of analyte is applied thereto.

10. A device as claimed in any one of the preceding claims, further including a housing in which is located a reservoir of washing fluid, and means for releasing a metered quantity of washing fluid onto the area of the test member to which fluid to be tested is to be applied.

11. A device as claimed in any one of the preceding claims, wherein the test member (4) is permanently attached to the meter (5).

12. A device as claimed in any one of the preceding claims, wherein the test member (4) includes an immobilised enzyme reagent covered by a semi-permeable membrane.

13. A device as claimed in Claim 12, wherein the membrane comprises PVC.

14. A device as claimed in Claim 13, wherein the PVC is low molecular weight PVC.

15. A device as claimed in any one of Claims 12 to 14, wherein the membrane incorporates a surfactant.

16. A device as claimed in any one of Claims 12 to 15, wherein the enzyme reagent is immobilised on platinised carbon.

17. A device as claimed in Claim 16, wherein the platinised carbon is prepared from the deposition of colloidal platinum onto the surface of activated carbon particles by oxidative decomposition of a platinum salt.

18. A device as claimed in any one of Claims 12 to 17, wherein the enzyme reagent is glucose oxidase or glucose dehydrogenase.

19. A device as claimed in any one of the preceding claims, further provided with means for audibly warning a user when a reading of analyte concentration is too low or too high, outside a defined pre-programmed concentration range.

20. A device as claimed in any one of the preceding claims, wherein the test member comprises a test strip contained within a pre-formed plastic casing.

21. A device as claimed in Claim 20, wherein the plastic casing and the test strip will have a void volume of less than 10 µl.

22. A device as claimed in Claim 20 or Claim 21, wherein the plastic casing has an aperture at the furthest extremity of the strip, to allow biological fluid to flow to an electrode surface on the strip by capillary forces.

23. A device as claimed in any one of claims 20 to 22, the casing further being provided, at an end of the plastic casing adjacent the meter (5), with two nipples, one attached to a washing fluid reservoir and the other attached to a calibration solution reservoir, both reservoirs being located within a housing (12) associated with the meter (5).

24. A test kit for point of care testing of analyte concentration in a fluid, the kit comprising a device (2) as claimed in Claim 7, and a container containing a control solution for use in calibrating the meter (5).

25. A device as claimed in claim 12 wherein the test member (4) comprises a printed layer of an ink comprising platinised carbon on which has been adsorbed at least one suitable enzyme reagent, a polyester resin binder, and a carrier fluid.

26. A cartridge (12) for use in the manufacture of a device as claimed in Claim 8, comprising a test member (4) provided with sensing means adapted to produce an electrical signal in response to the concentration of analyte in an applied fluid, and at least some of the electronics means of a meter (5), the cartridge (12) having means for releasable connection to a housing (16) which houses display means (6) for displaying an output reading which is dependent on the electrical signal; **characterised in that** the test member (4) is re-usable and permanently attached to the at least some of the electronics means of the meter (5).

27. A cartridge (12) as claimed in Claim 26, wherein the test member (4) is permanently attached to the meter (5).

28. A cartridge (12) as claimed in Claim 26 or Claim 27, wherein the electrical signal from the sensing means is an electric current.

## Patentansprüche

1. Vorrichtung (2) zur Grenzwertanalyse einer Analytkonzentration in einer auf diese aufgebrachten Flüssigkeit, wobei die Vorrichtung ein mit einer Erfassungseinrichtung ausgestattetes Testelement (4) umfasst, das dazu ausgelegt ist, im Ansprechen auf die Analytkonzentration in einer aufgebrachten Flüssigkeit ein elektrisches Signal zu erzeugen, und ein Messgerät (5), das eine elektronische Einrichtung aufweist, um ein Ausgangssignal zu erzeugen, das von dem elektrischen Signal aus dem Testelement (4) abhängt, wobei das Messgerät (5) mit einer Anzeigeeinrichtung (6) ausgestattet ist, um das Ausgangssignal anzuzeigen,
**dadurch gekennzeichnet, dass**
das Testelement (4) wiederverwendbar und dauerhaft bezüglich zumindest eines Teils des Messgeräts (5) befestigt ist.

2. Vorrichtung nach Anspruch 1, bei der die Erfassungseinrichtung ein Reagens oder mehrere Reagenzien umfasst, das/die mit dem Analyt reagiert/reagieren, um zumindest eine elektroreaktive Spezies zu erzeugen, und eine elektrische Schaltung, die einen Strom fließen lässt, dessen Größenordnung je nach der Konzentration des zugeführten Analyts variiert.

3. Vorrichtung nach Anspruch 1 oder 2, bei der das Testelement (4) mit einer Kappe (10) ausgestattet ist, die den Bereich abdeckt, auf die die Flüssigkeit aufgebracht werden soll, wobei die Innenoberfläche der Kappe (10) mit Einrichtungen ausgestattet ist, um das Testelement (4) abzuwischen oder zu waschen, wenn die Kappe vom Testelement abgenommen und/oder wieder auf dieses aufgesetzt wird.

4. Vorrichtung nach Anspruch 3, darüber hinaus eine Zähleinrichtung umfassend, die jeden Fall aufzeichnet, in dem die Kappe (10) vom Testelement (4) abgenommen und/oder wieder auf dieses aufgesetzt wird.

5. Vorrichtung nach Anspruch 4, die darüber hinaus Arretiereinrichtungen, um die Kappe (10) am Testelement (4) zu arretieren, und Einrichtungen, um die Arretiereinrichtungen zu aktivieren, wenn die Zähleinrichtung eine vorbestimmte Zahl aufzeichnet, umfasst.

6. Vorrichtung nach einem der vorhergehenden Ansprüche, bei der die Anzeigeeinrichtung (6) mehrere Leuchtdioden umfasst, die jeweils einer daneben angeordneten gedruckten Skala (8) entsprechen.

7. Vorrichtung nach einem der vorhergehenden Ansprüche, bei der das Messgerät (5) elektronische Abgleicheinrichtungen umfasst, um das Ausgangssignal aus dem Messgerät (5) im Ansprechen auf das elektrische Signal aus dem Testelement (4) abzugleichen, wenn diesem eine bekannte Konzentration an Analyt zugeführt wird.

8. Vorrichtung nach einem der vorhergehenden Ansprüche, bei der mindestens ein Teil der elektronischen Einrichtung des Messgeräts (5) dauerhaft bezüglich des Testelements (4) befestigt und in einer Hülse (12) untergebracht sind, die lösbar an einem Gehäuse (16) befestigt ist, das die Anzeigeeinrichtung (6) trägt.

9. Vorrichtung nach Anspruch 8, bei der die in der Hülse (12) untergebrachte elektronische Einrichtung Abgleicheinrichtungen umfasst, um das Ausgangssignal aus dem Messgerät (5) im Ansprechen auf das elektrische Signal aus dem Testelement (4) abzugleichen, wenn auf dieses eine bekannte Konzentration an Analyt aufgebracht wird.

10. Vorrichtung nach einem der vorhergehenden Ansprüche, die darüber hinaus ein Gehäuse umfasst, in dem sich ein Tank für Waschflüssigkeit befindet, und eine Einrichtung, um eine dosierte Menge an Waschflüssigkeit auf den Bereich des Testelements abzugeben, auf den Flüssigkeit aufgebracht werden soll, die zu analysieren ist.

11. Vorrichtung nach einem der vorhergehenden Ansprüche, bei der das Testelement (4) dauerhaft am Messgerät (5) befestigt ist.

12. Vorrichtung nach einem der vorhergehenden Ansprüche, bei der das Testelement (4) ein Reagens aus immobilisiertem Enzym umfasst, das von einer semi-permeablen Membran bedeckt ist.

13. Vorrichtung nach Anspruch 12, bei der die Membran PVC umfasst.

14. Vorrichtung nach Anspruch 13, bei der das PVC ein PVC niedrigen Molekulargewichts ist.

15. Vorrichtung nach einem der Ansprüche 12 bis 14, bei der die Membran einen oberflächenaktiven Stoff beinhaltet.

16. Vorrichtung nach einem der Ansprüche 12 bis 15, bei der das Enzymreagens auf Platinkohle immobilisiert ist.

17. Vorrichtung nach Anspruch 16, bei der die Platinkohle aus der Ablagerung kolloidalen Platins auf der Oberfläche von Aktivkohlepartikeln durch oxidativen Zersetzung eines Platinsalzes hergestellt wird.

18. Vorrichtung nach einem der Ansprüche 12 bis 17, bei der das Enzymreagens Glucoseoxidase oder Glucosedehydrogenase ist.

19. Vorrichtung nach einem der vorhergehenden Ansprüche, die darüber hinaus mit einer Einrichtung ausgestattet ist, um einen Benutzer akustisch zu warnen, wenn ein Skalenwert der Analytkonzentration außerhalb eines definierten vorprogrammierten Konzentrationsbereichs zu niedrig oder zu hoch ist.

20. Vorrichtung nach einem der vorhergehenden Ansprüche, bei der das Testelement einen in einem vorgeformten Kunststoffgehäuse enthaltenen Teststreifen umfasst.

21. Vorrichtung nach Anspruch 20, bei der das Kunststoffgehäuse und der Teststreifen ein Hohlraumvolumen von weniger als 10 µl haben.

22. Vorrichtung nach Anspruch 20 oder 21, bei der das Kunststoffgehäuse am äußersten Ende des Streifens eine Öffnung besitzt, damit eine biologische Flüssigkeit durch Kapillarkräfte zu einer Elektrodenoberfläche am Streifen fließen kann.

23. Vorrichtung nach einem der Ansprüche 20 bis 22, wobei das Gehäuse an einem Ende des Kunststoffgehäuses neben dem Messgerät (5) noch mit zwei Nippeln ausgestattet ist, von denen einer an einem Waschflüssigkeitstank befestigt ist, und der andere an einem Abgleichlösungstank befestigt ist, wobei sich beide Tanks innerhalb eines Gehäuses (12) befinden, das mit dem Messgerät (5) verbunden ist.

24. Test-Kit zur Grenzwertanalyse der Analytkonzentration in einer Flüssigkeit, wobei das Kit eine Vorrichtung (2) nach Anspruch 7 und einen Behälter umfasst, der eine Kontrolllösung zur Verwendung beim Abgleich des Messgeräts (5) enthält.

25. Vorrichtung nach Anspruch 12, bei der das Testelement (4) eine gedruckte Schicht einer Druckfarbe aufweist, die Platinkohle umfasst, auf die mindestens ein geeignetes Enzymreagens, ein Polyesterharzbinder und eine Trägerflüssigkeit adsorbiert wurde.

26. Hülse (12) zur Verwendung bei der Herstellung einer Vorrichtung nach Anspruch 8, die ein Testelement (4) umfasst, das mit einer Erfassungseinrichtung ausgestattet ist, die dazu ausgelegt ist, ein elektrisches Signal im Ansprechen auf die Analytkonzentration in einer aufgebrachten Flüssigkeit zu erzeugen, und zumindest einen Teil der elektronischen Einrichtung eines Messgeräts (5), wobei die Hülse (12) Einrichtungen zur lösbaren Verbindung mit einem Gehäuse (16) aufweist, in dem eine Anzeigeeinrichtung (6) zur Anzeige eines Ausgangsskalenwerts untergebracht ist, der von dem elektrischen Signal abhängt;
**dadurch gekennzeichnet, dass** das Testelement (4) wiederverwendbar und dauerhaft an zumindest einem Teil der elektronischen Einrichtung des Messgeräts (5) befestigt ist.

27. Hülse (12) nach Anspruch 26, bei der das Testelement (4) dauerhaft am Messgerät (5) befestigt ist.

28. Hülse (12) nach Anspruch 26 oder 27, bei der das elektrische Signal aus der Erfassungseinrichtung ein elektrischer Strom ist.

## Revendications

1. Un dispositif (2) pour tester au moment de soins la concentration d'analyte dans un fluide qui lui est appliqué, le dispositif comprenant un élément de test (4) muni d'un dispositif de détection agencé pour produire un signal électrique en réponse à la concentration d'analyte dans un fluide appliqué et un dispositif de mesure (5) comprenant des moyens électroniques pour produire une sortie qui est fonction du signal électrique de l'élément de test (4), le dispositif de mesure (5) étant muni d'un dispositif d'affichage (6) pour afficher la sortie du signal, **caractérisé en ce que** l'élément de test (4) est réutilisable et fixé de façon permanente par rapport à au moins une partie du dispositif de mesure (5).

2. Un dispositif selon la revendication 1, dans lequel le moyen de détection comprend un ou plusieurs réactifs qui réagissent avec l'analyte pour produire au moins une espèce électroactive, et un circuit électrique qui fait passer du courant dont la grandeur varie conformément à la concentration de l'analyte appliqué.

3. Un dispositif selon la revendication 1 ou la revendication 2, dans lequel l'élément de test (4) est muni d'un capuchon (10) qui recouvre la zone à laquelle le fluide doit être appliqué, la surface interne du capuchon (10) étant munie d'un dispositif pour essuyer ou laver l'élément de test (4) lorsque le capuchon est retiré de l'élément de test et/ou replacé sur l'élément de test.

4. Un dispositif selon la revendication 3, comprenant, en outre, un compteur qui enregistre chaque fois lorsque le capuchon (10) est retiré de l'élément de test (4) et/ou replacé sur celui-ci.

5. Un dispositif selon la revendication 4, comprenant, en outre, un moyen de verrouillage pour verrouillé le capuchon (10) sur l'élément de teste (4) et moyen pour actionner le moyen de verrouillage lorsque le compteur enregistre un nombre prédéterminé.

6. Un dispositif selon l'une quelconque des revendications précédentes, dans lequel le moyen d'affichage (6) comprend une pluralité de diodes émettant de la lumière, dont chacune correspond à une échelle imprimée contiguë (8).

7. Un dispositif selon l'une quelconque des revendications précédentes, dans lequel le dispositif de mesure (5) comprend un moyen d'étalonnage électronique pour étalonner la sortie de signal à partir du dispositif de mesure (5) en réponse au signal électrique à partir de l'élément de test (4) lorsqu'une concentration connue d'analyte lui est appliquée.

8. Un dispositif selon l'une quelconque des revendications précédentes, dans lequel au moins certains des moyens électroniques du dispositif de mesure (5) sont fixés de façon permanente par rapport à l'élément de test (4) et sont logés dans une cartouche (12) qui est fixée de façon amovible à un logement (16) qui supporte le moyen d'affichage (6).

9. Un dispositif selon la revendication 8, dans lequel les moyens électroniques logés dans la cartouche (12) comportent un moyen d'étalonnage pour étalonner la sortie de signal à partir du dispositif de mesure (5) en réponse au signal électrique à partir de l'élément de test (4) lorsqu'une concentration connue d'analyte lui est appliquée.

10. Un dispositif selon l'une quelconque des revendications précédentes, comprenant, en outre, un logement dans lequel est disposé un réservoir de fluide de lavage, et un moyen pour libérer une quantité dosée de fluide de lavage sur la zone de l'élément de test auquel le fluide à éprouver doit être appliqué.

11. Un dispositif selon l'une quelconque des revendications précédentes, dans lequel l'élément de test (4) est fixé de façon permanente au dispositif de mesure (5).

12. Un dispositif selon l'une quelconque des revendications précédentes, dans lequel l'élément de test (4) comprend un réactif d'enzyme immobilisé recouvert par une membrane semi-perméable.

13. Un dispositif selon la revendication 12, dans lequel la membrane comprend du PVC.

14. Un dispositif selon la revendication 13, dans lequel le PVC est du PVC de bas poids moléculaire.

15. Un dispositif selon l'une quelconque des revendications 12 à 14, dans lequel la membrane renferme un tensioactif.

16. Un dispositif selon l'une quelconque des revendications 12 à 15, dans lequel le réactif d'enzyme est immobilisé sur du carbone platiné.

17. Un dispositif selon la revendication 16, dans lequel le carbone platiné est préparé à partir du dépôt de platine colloïdal sur la surface des particules de carbone activé par décomposition oxydante d'un sel de platine.

18. Un dispositif selon l'une quelconque des revendications 12 à 17, dans lequel le réactif d'enzyme est la glucose oxydase ou la glucose deshydrogénase.

19. Un dispositif selon l'une quelconque des revendications précédentes, muni, en outre, d'un moyen pour indiquer de façon sonore à un utilisateur lorsqu'une lecture de la concentration d'analyte est trop faible ou trop élevée, en dehors d'une gamme de concentrations préprogrammée définie.

20. Un dispositif selon l'une quelconque des revendications précédentes, dans lequel l'élément de test comprend une bande de test contenue dans une enveloppe en matière plastique préformée.

21. Un dispositif selon la revendication 20, dans lequel l'enveloppe de matière plastique et la bande de test ont un volume de vide inférieur à 10 µl.

22. Un dispositif selon la revendication 20 ou la revendication 21, dans lequel l'enveloppe en matière plastique présente une ouverture à l'extrémité la plus éloignée de la bande pour permettre au fluide biologique de s'écouler vers une surface d'électrode sur la bande par des forces capillaires.

23. Un dispositif selon l'une quelconque des revendications 20 à 22, l'enveloppe étant munie, en outre, à une extrémité de l'enveloppe en matière plastique contiguë au dispositif de mesure (5), de deux raccords, l'un étant fixé à un réservoir de fluide de lavage et l'autre étant fixé à un réservoir de solution d'étalonnage, les deux réservoirs étant disposés dans un logement (12) associé au dispositif de mesure (5).

24. Un nécessaire de test pour tester au moment de soin la concentration d'analyte dans un fluide, le nécessaire comprenant un dispositif (2), comme revendiqué dans la revendication 7, et un récipient renfermant une solution témoin destinée à être utilisée dans l'étalonnage du dispositif de mesure (5).

25. Un dispositif selon la revendication 12, dans lequel l'élément de test (4) comprend une couche imprimée d'une encre comprenant du carbone platiné sur lequel a été adsorbé au moins un réactif d'enzyme approprié, un liant de résine de polyester et un fluide véhicule.

26. Une cartouche (12) destinée à être utilisée dans la fabrication d'un dispositif selon la revendication 8, comprenant un élément de test (4) muni d'un moyen de détection agencé pour produire un signal électrique en réponse à la concentration d'analyte dans un fluide appliqué, et au moins certains moyens électroniques d'un dispositif de mesure (5), la cartouche (12) ayant des moyens pour une liaison libérable à un logement (16), lequel loge les moyens d'affichage (6) pour afficher une lecture de sortie qui est fonction du signal électrique, **caractérisée en ce que** l'élément de test (4) est réutilisable et fixé de façon permanente à au moins certains des moyens électroniques du dispositif de mesure (5).

27. Une cartouche (12) selon la revendication 26, dans laquelle l'élément de test (4) est fixé en permanence au dispositif de mesure (5).

28. Une cartouche (12) selon la revendication 26 ou la revendication 27, dans laquelle le signal électrique à partir du moyen de détection est un courant électrique.
